# EUROPEAN PATENT APPLICATION

(11) **EP 3 023 153 A2**
(43) Date of publication of application: **25.05.2016**
(21) Application number: 15195649.7
(22) Date of filing: 20.11.2015
(51) Int. Cl.: B01L 7/00

(54) **NUCLEIC ACID AMPLIFICATION REACTION APPARATUS AND NUCLEIC ACID AMPLIFICATION METHOD**

(30) Priority: 20.11.2014 JP 2014235322
(71) Applicant: Seiko Epson Corporation, Shinjuku-ku Tokyo 163-0811 (JP)
(72) Inventor: Uehara, Masayuki, Nagano, 390-8621 (JP); Idegami, Kotaro, Nagano, 392-8502 (JP); Hanamura, Masato, Nagano, 392-8502 (JP); Saito, Yuji, Nagano, 392-8502 (JP); Yamaguchi, Akemi, Nagano, 392-8502 (JP)
(74) Representative: Hoffmann Eitle

(57) **Abstract**

A nucleic acid amplification reaction apparatus includes: a fitting section capable of fitting a nucleic acid amplification reaction vessel filled with a nucleic acid amplification reaction mixture and a liquid which has a specific gravity different from that of the nucleic acid amplification reaction mixture and is immiscible with the nucleic acid amplification reaction mixture; a first heating section which heats a first region of the nucleic acid amplification reaction vessel to a first temperature; a second heating section which heats a second region of the nucleic acid amplification reaction vessel to a second temperature; and a driving mechanism which switches over between a first arrangement in which the first region is located lower than the second region in the direction of the gravitational force and a second arrangement in which the second region is located lower than the first region in the direction of the gravitational force, wherein the concentration of magnesium ions in the nucleic acid amplification reaction mixture is 1.8 mM or more and 9.0 mM or less.

## Description

### BACKGROUND

### 1. Technical Field

The present invention relates to a nucleic acid amplification reaction apparatus and a nucleic acid amplification method.

### 2. Related Art

In recent years, as a result of development of technologies utilizing genes, medical treatments utilizing genes such as gene diagnosis or gene therapy have been drawing attention. In addition, many methods using genes in determination of breed varieties or breed improvement have also been developed in the field of agriculture and livestock. As technologies for utilizing genes, nucleic acid amplification technologies such as a PCR (Polymerase Chain Reaction) method have been widely used. In recent years, a PCR apparatus capable of obtaining a nucleic acid amplification reaction product in a short time has been developed (for example, JP-A-2012-115208 (PTL1)). It is important to set the concentration of magnesium chloride in the preparation of a nucleic acid amplification reaction reagent, and it has been recognized that when the concentration of magnesium chloride is low, the yield decreases, and when the concentration of magnesium chloride is high, the nucleic acid amplification reaction is inhibited or non-specific amplification can be caused. In many known commercially available products, the recommended concentration of magnesium chloride in a reaction mixture with which nucleic acid amplification is performed is 1.5 mM (see, for example, a website for troubleshooting regarding PCR of TOYOBO CO., LTD. Life Science Department (http://www.toyobo.co.jp/seihin/xr/lifescience/products/pr oduct/jisshirei/archives/2008/04/post_21.html) (NPL 1) and page 6 of the operation manual for TaqDNA Polymerase and Platinum Taq antibody of Life Technologies, Inc. (http://tools.lifetechnologies.com/content/sfs/productnote s/f_050711_taq-ts-tl-mkt-hl.pdf) (NPL 2)).

### SUMMARY

The present inventors found that in a nucleic acid amplification reaction using the above-mentioned PCR apparatus, when a reaction time, particularly, an annealing time or an annealing and elongation time is decreased, a sufficient amount of a nucleic acid amplification reaction product cannot be obtained as compared with the case where a reaction time is long. Accordingly, an advantage of some aspects of the invention is to provide a nucleic acid amplification reaction apparatus and a nucleic acid amplification method capable of obtaining a sufficient amount of a nucleic acid amplification reaction product even in the case where a reaction time is short.

The invention can be implemented as the following forms.

A nucleic acid amplification reaction apparatus according to an aspect of the invention includes: a fitting section capable of fitting a nucleic acid amplification reaction vessel filled with a nucleic acid amplification reaction mixture and a liquid which has a specific gravity smaller than that of the nucleic acid amplification reaction mixture and is immiscible with the nucleic acid amplification reaction mixture; a first heating section which heats a first region of the nucleic acid amplification reaction vessel to a first temperature; a second heating section which heats a second region of the nucleic acid amplification reaction vessel to a second temperature; and a driving mechanism which switches over between a first arrangement in which the first region is located lower than the second region in the direction of the gravitational force and a second arrangement in which the second region is located lower than the first region in the direction of the gravitational force, wherein the concentration of magnesium ions in the nucleic acid amplification reaction mixture is 1.8 mM or more and 9.0 mM or less. The concentration of magnesium ions in the nucleic acid amplification reaction mixture may be 2.0 mM or more and 7.5 mM or less. The nucleic acid amplification reaction apparatus may further include a control section which controls the driving mechanism, and a period in which the control section maintains the first arrangement may be 4.5 seconds or less, and a period in which the control section maintains the second arrangement may be 18 seconds or less. A period in which the control section maintains the first arrangement may be 4 seconds or less. A period in which the control section maintains the second arrangement may be 6 seconds or less.

A nucleic acid amplification reaction cartridge according to another aspect of the invention includes a tube, a nucleic acid amplification reaction vessel, and a plunger. The tube is internally provided, in the following order, with a first plug composed of a first oil, a second plug composed of a washing liquid, which is immiscible with an oil, and with which a nucleic acid-binding solid-phase carrier having a nucleic acid bound thereto is washed, a third plug composed of a second oil, a fourth plug composed of an eluent, which is immiscible with an oil, and with which the nucleic acid is eluted from the nucleic acid-binding solid-phase carrier having the nucleic acid bound thereto, a fifth plug composed of a third oil, a sixth plug composed of a nucleic acid amplification reaction mixture, which is immiscible with an oil, and with which a nucleic acid amplification reaction is performed, and a seventh plug composed of a fourth oil. The nucleic acid amplification reaction vessel communicates with the tube on the seventh plug side and contains an oil. The plunger is attached to an opening section of the tube on the first plug side so as to push out the liquid from the tube on the seventh plug side to the nucleic acid amplification reaction vessel. The concentration of magnesium ions in a mixed liquid of the eluent and the nucleic acid amplification reaction mixture is 1.8 mM or more and 9.0 mM or less.

A nucleic acid amplification reaction cartridge according to still another aspect of the invention includes a tube, a nucleic acid amplification reaction vessel, and a plunger. The tube is internally provided, in the following order, with a first plug composed of a first oil, a second plug composed of a washing liquid, which is immiscible with an oil, and with which a nucleic acid-binding solid-phase carrier having a nucleic acid bound thereto is washed, a third plug composed of a second oil, a fourth plug composed of an eluent, which is immiscible with an oil, and with which the nucleic acid is eluted from the nucleic acid-binding solid-phase carrier having the nucleic acid bound thereto, and a fifth plug composed of a third oil. The nucleic acid amplification reaction vessel communicates with the tube on the fifth plug side and contains an oil. The plunger is attached to an opening section of the tube on the first plug side so as to push out the liquid from the tube on the fifth plug side to the nucleic acid amplification reaction vessel. The nucleic acid amplification reaction vessel contains a liquid droplet composed of a nucleic acid amplification reaction mixture, which is immiscible with an oil, and with which a nucleic acid amplification reaction is performed. The concentration of magnesium ions in a mixed liquid of the eluent and the nucleic acid amplification reaction mixture is 1.8 mM or more and 9.0 mM or less.

A nucleic acid amplification reaction cartridge according to yet another aspect of the invention includes a tube, a nucleic acid amplification reaction vessel, and a plunger. The tube is internally provided, in the following order, with a first plug composed of a first oil, a second plug composed of a washing liquid, which is immiscible with an oil, and with which a nucleic acid-binding solid-phase carrier having a nucleic acid bound thereto is washed, a third plug composed of a second oil, a fourth plug composed of an eluent, which is immiscible with an oil, and with which the nucleic acid is eluted from the nucleic acid-binding solid-phase carrier having the nucleic acid bound thereto, and a fifth plug composed of a third oil. The nucleic acid amplification reaction vessel communicates with the tube on the fifth plug side and contains an oil. The plunger is attached to an opening section of the tube on the first plug side so as to push out the liquid from the tube on the fifth plug side to the nucleic acid amplification reaction vessel. The concentration of magnesium ions in the eluent is 1.8 mM or more and 9.0 mM or less.

A nucleic acid amplification method according to still yet another aspect of the invention includes: heating a first region of a nucleic acid amplification reaction vessel filled with a nucleic acid amplification reaction mixture and a liquid which has a specific gravity smaller than that of the nucleic acid amplification reaction mixture and is immiscible with the nucleic acid amplification reaction mixture to a first temperature; heating a second region of the nucleic acid amplification reaction vessel to a second temperature lower than the first temperature; maintaining a first arrangement in which the first region is located lower than the second region in the direction of the gravitational force for a first period so as to allow the nucleic acid amplification reaction mixture to move into the first region; and maintaining a second arrangement in which the second region is located lower than the first region in the direction of the gravitational force for a second period so as to allow the nucleic acid amplification reaction mixture to move into the second region, wherein the concentration of magnesium ions in the nucleic acid amplification reaction mixture is 1.8 mM or more and 9.0 mM or less. The first period may be 4.5 seconds or less, and the second period may be 18 seconds or less.

A nucleic acid amplification method according to further another aspect of the invention includes performing a cycle at predetermined times, the cycle including: maintaining a nucleic acid amplification reaction mixture in a first region at a first temperature of a liquid which is immiscible with the nucleic acid amplification reaction mixture for 4.5 seconds or less; allowing the nucleic acid amplification reaction mixture to move into a second region at a second temperature lower than the first temperature; and maintaining the nucleic acid amplification reaction mixture in the second region for 18 seconds or less, wherein the concentration of magnesium ions in the nucleic acid amplification reaction mixture is 1.8 mM or more and 9.0 mM or less.

According to the aspects of the invention, a novel nucleic acid amplification reaction apparatus and a novel nucleic acid amplification method can be provided.

### BRIEF DESCRIPTION OF THE DRAWINGS

The invention will be described with reference to the accompanying drawings, wherein like numbers reference like elements.
FIG. 1 is a cross-sectional view of a nucleic acid amplification reaction vessel according to one embodiment of the invention. The arrow g indicates the direction of the gravitational force.
FIGS. 2A and 2B are perspective views of an up-and-down type PCR apparatus according to one embodiment of the invention. FIG. 1A shows a state in which a lid is closed and FIG. 1B shows a state in which the lid is opened.
FIG. 3 is an exploded perspective view of a main body of the up-and-down type PCR apparatus according to one embodiment of the invention.
FIGS. 4A and 4B are cross-sectional views schematically showing the cross section taken along the line A-A of FIG. 2A of the main body of the up-and-down type PCR apparatus according to one embodiment of the invention. FIG. 4A shows a first arrangement and FIG. 4B shows a second arrangement.
FIGS. 5A and 5B are explanatory views of a cartridge according to one embodiment of the invention.
FIG. 6 is a graph showing the relationship between the concentration of magnesium ions and the amount of a nucleic acid amplification reaction product.
FIG. 7 is a graph showing the relationship between the concentration of magnesium ions and the amount of a nucleic acid amplification reaction product.
FIG. 8 is a graph showing the relationship between the concentration of potassium ions and the amount of a nucleic acid amplification reaction product.
FIG. 9 is a graph showing the relationship between the concentration of potassium ions and the amount of a nucleic acid amplification reaction product.
FIG. 10 is a graph showing the relationship between the concentration of magnesium ions and the amount of a nucleic acid amplification reaction product.
FIG. 11 is a graph showing the relationship between the concentration of magnesium ions and the amount of a nucleic acid amplification reaction product.
FIG. 12 is a view showing the presence or absence of a specific amplification product and a non-specific amplification product in Example of the invention and Comparative Example when the concentration of magnesium ions in a nucleic acid amplification reaction mixture was 5 mM.
FIG. 13 is a view showing the presence or absence of a specific amplification product and a non-specific amplification product in Example of the invention and Comparative Example when the concentration of magnesium ions in a nucleic acid amplification reaction mixture was 5 mM.

### DESCRIPTION OF EXEMPLARY EMBODIMENTS

The object, features, and advantages of the invention as well as the idea thereof will be apparent to those skilled in the art from the description given herein, and the invention can be easily reproduced by those skilled in the art based on the description given herein. It is to be understood that the embodiments, specific examples, etc. of the invention described below are to be taken as preferred embodiments of the invention, and are presented for illustrative or explanatory purposes and are not intended to limit the invention. It is further apparent to those skilled in the art that various changes and modifications may be made based on the description given herein within the intent and scope of the invention disclosed herein.

### (1) Nucleic Acid Amplification Reaction Vessel

A nucleic acid amplification reaction vessel to be used in the method according to the invention is a nucleic acid amplification reaction vessel which has a hermetically sealed vessel containing a reaction mixture and a liquid which has a specific gravity different from that of the reaction mixture and is phase-separated from the reaction mixture, wherein the reaction mixture is in the form of a liquid droplet and the liquid contains an oil and an additive.

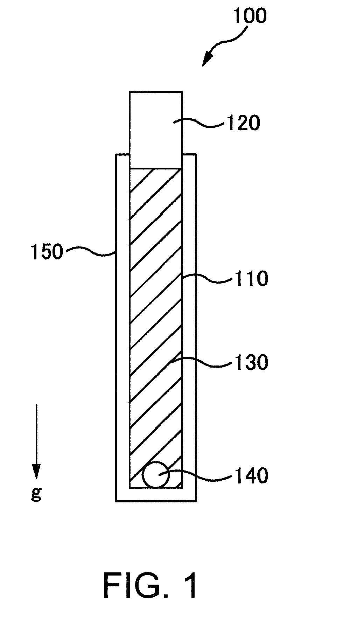

FIG. 1 is a cross-sectional view of a nucleic acid amplification reaction vessel 100. FIG. 1 shows a state in which a reaction mixture is placed in the nucleic acid amplification reaction vessel.

The nucleic acid amplification reaction vessel 100 to be used in the invention is configured to include a vessel 150 and a sealing section 120 . The size and shape of the nucleic acid amplification reaction vessel 100 are not particularly limited, but may be designed in consideration of, for example, at least one of the amount of a liquid 130 which is immiscible with a reaction mixture 140, the thermal conductivity thereof, the shapes of the vessel 150 and the sealing section 120, and the ease of handling thereof.

The vessel 150 of the nucleic acid amplification reaction vessel 100 can be formed from a transparent material. According to this, the movement of the reaction mixture 140 in the vessel 150 can be observed from the outside of the nucleic acid amplification reaction vessel 100, or the vessel 150 can be used in an application in which the measurement or the like is performed from the outside of the vessel 150 such as real-time PCR. The term "transparent" as used herein refers to a condition in which the visibility can be ensured to such an extent that the reaction mixture 140 in the vessel 150 can be observed from the outside of the vessel 150, and it is not necessary that the entire nucleic acid amplification reaction vessel 100 should be transparent as long as this condition is met.

The application of the nucleic acid amplification reaction vessel 100 is not particularly limited, however, for example, in the case where the nucleic acid amplification reaction vessel 100 is used in an application with a fluorescence measurement such as real-time PCR, the vessel 150 is desirably formed from a material with a low autofluorescence. The vessel 150 is preferably formed from a material which can withstand heating in PCR. Further, the material of the vessel 150 is preferably a material, on which nucleic acids or proteins are less adsorbed, and which does not inhibit the enzymatic reaction by a polymerase or the like. The material which satisfies these conditions is not particularly limited, and for example, polypropylene, polyethylene, a cycloolefin polymer (for example, ZEONEX (registered trademark) 480R), a heat-resistant glass (for example, PYREX (registered trademark) glass), or the like, or a composite material thereof may be used, however, polypropylene is preferred.

In the nucleic acid amplification reaction vessel 100 shown in FIG. 1, the vessel 150 is formed into a cylindrical shape, and the direction of the center axis (the vertical direction in Fig. 1) coincides with the longitudinal direction. The vessel 150 used here is preferably a tube and may be a tube for a microcentrifuge or a tube designed for PCR. Since the vessel 150 has a shape with a longitudinal direction, in other words, an elongated shape, for example, in the case where the temperature of the nucleic acid amplification reaction vessel 100 is controlled so that regions having different temperatures are formed in the liquid 130 in the vessel 150 using an up-and-down type thermal cycler, which will be described later, the distance between the regions having different temperatures is easily increased, According to this, it becomes easy to control the temperature of the liquid 130 to be different from region to region in the vessel 150, and therefore, a thermal cycle suitable for PCR can be realized. The "up-and-down type thermal cycler" is an apparatus which realizes a thermal cycle by forming at least two temperature regions in a liquid filled in the vessel 150 and allowing the reaction mixture 140 which is phase-separated from the liquid to move reciprocatingly between these temperature regions.

The shape of the vessel 150 is not particularly limited as long as it has a longitudinal direction, however, in the case where the vessel 150 is used for an up-and-down type PCR apparatus, it is preferred that the shape is a substantially cylindrical shape and the ratio of the inner diameter D to the length L in the longitudinal direction is in the range of 1:5 to 5:20. It is more preferred that the inner diameter D is from 1.5 to 2 mm, and the length L is from 10 to 20 mm.

The vessel 150 has an opening section and the sealing section 120 which seals the opening section, and in the vessel 150, the reaction mixture 140 and the liquid 130 which has a specific gravity different from that of the reaction mixture 140 and is phase-separated from the reaction mixture 140 are contained. It is preferred that in the case where the opening section is sealed by the sealing section 120, air does not remain in the vessel 150 . It is because if an air bubble remains in the vessel 150, the movement of the reaction mixture 140 may be hindered. The sealing section 120 can be formed from the same material as that of the vessel 150. The structure of the sealing section 120 may be any as long as it can hermetically seal the vessel 150, and can be a structure of, for example, a screw cap, a plug, an inlay, or the like. In FIG. 1, the sealing section 120 has a structure of a screw cap.

The nucleic acid amplification reaction mixture 140 may contain a nucleic acid amplification reaction reagent and a target nucleic acid to be amplified. Examples of the target nucleic acid include a DNA prepared from a specimen such as blood, urine, saliva, spinal fluid, or a tissue and a cDNA obtained by reverse transcription of an RNA prepared from any of the above specimens. The nucleic acid amplification reaction reagent may contain a primer for amplifying a target nucleic acid, a buffer, a polymerase, dNTPs, MgCl₂, a fluorescent label for detecting an amplification product of the target nucleic acid, and the like. The DNA polymerase is not particularly limited, but is preferably a heat-resistant enzyme or an enzyme for use in PCR, and there are a great number of commercially available products, for example, Taq polymerase, Tfi polymerase, Tth polymerase, modified forms thereof, and the like, however, a DNA polymerase capable of performing hot start PCR is preferred. The concentration of dNTPs may be set to a concentration suitable for the enzyme to be used, however, the concentration of dNTPs may be set to generally 10 to 1000 µM, preferably 100 to 500 µM.

The concentration of magnesium ions (Mg²⁺) in the reaction mixture 140 is preferably 1.8 mM or more and 9.0 mM or less, more preferably 2.0 mM or more and 7.5 mM or less. The origin of Mg²⁺ is not particularly limited, and Mg²⁺ may be derived from magnesium chloride or magnesium sulfate, but is preferably derived from magnesium chloride.

The total ion concentration in the reaction mixture 140 is not particularly limited, but may be higher than 50 mM, and is preferably higher than 100 mM, more preferably higher than 120 mM, further more preferably higher than 150 mM, still further more preferably higher than 200 mM. The upper limit thereof is preferably 500 mM or less, more preferably 300 mM or less, further more preferably 200 mM or less. Each oligonucleotide for the primer is used at 0.1 to 10 µM, preferably at 0.1 to 1 µM.

The reaction mixture 140 may further contain a surfactant. The surfactant is not particularly limited, however, examples thereof include NP-40, Triton X-100, and Tween 20. The concentration of the surfactant is not particularly limited, but is preferably a concentration which does not inhibit the nucleic acid amplification reaction, and may be from 0.001% to 0.1% or less, and is preferably from 0.002% to 0.02%, and most preferably from 0.005% to 0.01%. The surfactant may be a carry-over from a stock solution of the enzyme described above, however, a surfactant solution may be added to the reaction mixture 140 independently of the stock solution of the enzyme.

By using a liquid which is immiscible with the reaction mixture 140 as the liquid 130, when the reaction mixture 140 is placed in the vessel 150, the reaction mixture 140 and the liquid 130 are phase-separated from each other, and therefore, the reaction mixture 140 can be formed into a liquid droplet in the liquid 130 . In this manner, the reaction mixture 140 is maintained in the form of a liquid droplet in the liquid 130.

The liquid 130 is preferably a liquid which has a specific gravity smaller than that of the reaction mixture 140. In this case, when the reaction mixture 140 is placed in the liquid 130, the liquid droplet of the reaction mixture 140 has a specific gravity larger than that of the liquid 130, and therefore moves in the direction of the gravitational force by the action of gravity. Further, the liquid 130 may be a liquid which has a specific gravity larger than that of the reaction mixture 140. In this case, the liquid droplet of the reaction mixture 140 has a specific gravity smaller than that of the liquid 130, and therefore moves in the direction opposite to the direction of the gravitational force by the action of gravity.

The liquid 130 preferably contains an oil, and for example, a silicone oil or a mineral oil can be used. Here, the "silicone" means an oiligomer or a polymer having a siloxane bond as a main skeleton. In this specification, among silicones, a silicone in the form of a liquid in a temperature range in which the silicone is used in a thermal cycling treatment is particularly referred to as "silicone oil". Further, in this specification, an oil which is purified from petroleum and is in the form of a liquid in a temperature range in which the oil is used in a thermal cycling treatment is referred to as "mineral oil". These oils have high stability against heat, and for example, products having a viscosity of 5 x 10³ Nsm⁻² or less are also easily available, and therefore, these oils are preferred for use in an up-and-down type PCR apparatus.

Examples of the silicone oil include dimethyl silicone oils such as KF-96L-0.65cs, KF-96L-lcs, KF-96L-2cs, KF-96L-5cs (manufactured by Shin-Etsu Silicone Co., Ltd.), SH200 C FLUID 5 CS (manufactured by Dow Corning Toray Co. , Ltd.), TSF451-5A, and TSF451-10 (manufactured by Momentive Performance Materials Japan LLC) . Examples of the mineral oil include oils containing alkane having about 14 to 20 carbon atoms as a principal component, and specific examples thereof include n-tetradecane, n-pentadecane, n-hexadecane, n-heptadecane, n-octadecane, n-nonadecane, and n-tetracosane.

The viscosity of the oil is not particularly limited, but is preferably 90 cs or less, more preferably 70 cs or less, further more preferably 50 cs or less, still further more preferably 30 cs or less. In this manner, the viscosity of the oil is preferably smaller, and according to this, when the reaction mixture precipitates as described later, the reaction mixture can precipitate more smoothly.

The liquid 130 may contain an additive. As the additive, for example, a modified silicone oil such as X-22-160AS, X-22-3701E, KF-857, KF-859, KF-862, KF-867, KF-6017, or KF-8005 (Shin-Etsu Silicone Co., Ltd.), a silicone resin such as SR1000, SS4230, SS4267, or YR3370 (Momentive Performance Materials, Inc.), a fluoro-modified silicone resin such as XS66-C1191 (Momentive Performance Materials, Inc.), or the like, and other than these, a modified silicone oil such as TSF4703, TSF4708, XF42-C5196, or XF42-C5197 (Momentive Performance Materials, Inc.) can be used. The concentration of the additive is not particularly limited, but can be determined in consideration of the structure, material, shape, or the like of the vessel. For example, the concentration thereof is preferably 1% (v/v) or more and 50% (v/v) or less, more preferably 2% (v/v) or more and 20% (v/v) or less, further more preferably 5% (v/v).

### (2) Configuration of Nucleic Acid Amplification Reaction Apparatus

In this embodiment, as the nucleic acid amplification reaction vessel to be used for performing a nucleic acid amplification reaction, a nucleic acid amplification reaction tube 100 in the form of a tube is used. Hereinafter, by taking PCR as one example of the nucleic acid amplification reaction, one example of a nucleic acid amplification reaction apparatus (hereinafter also referred to as "up-and-down type PCR apparatus") suitable for the nucleic acid amplification reaction tube 100 will be described in detail.

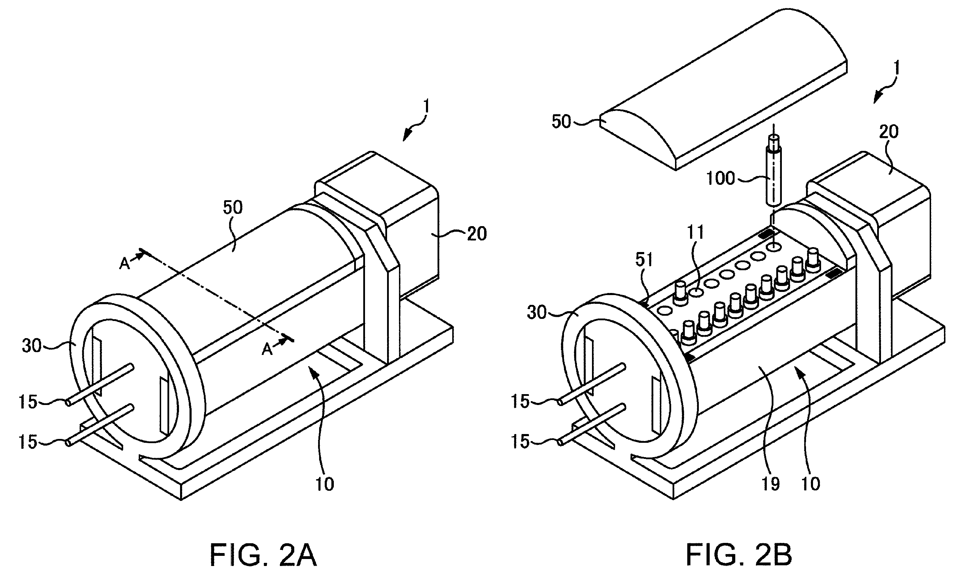

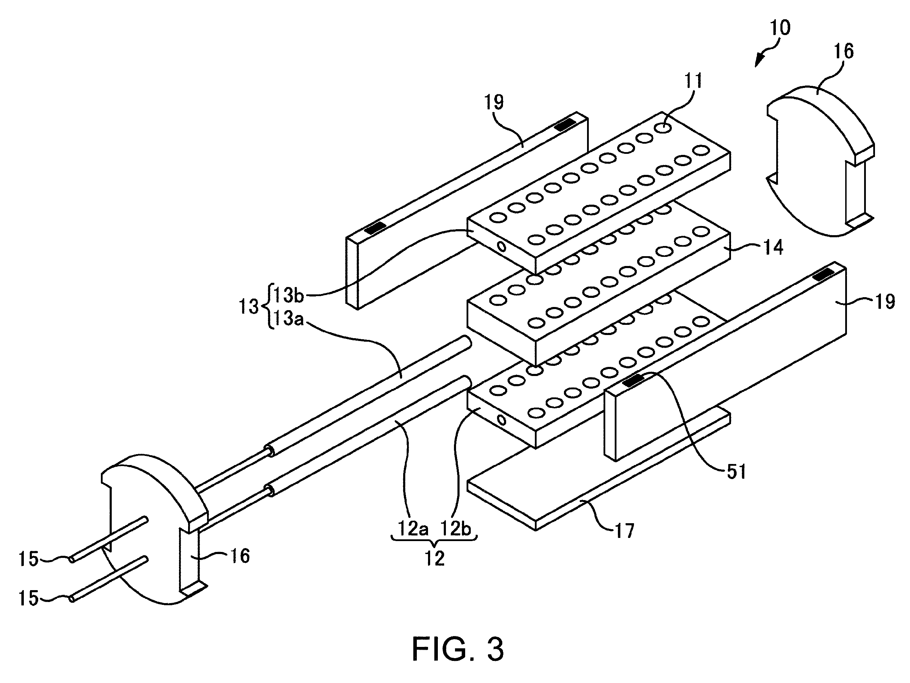

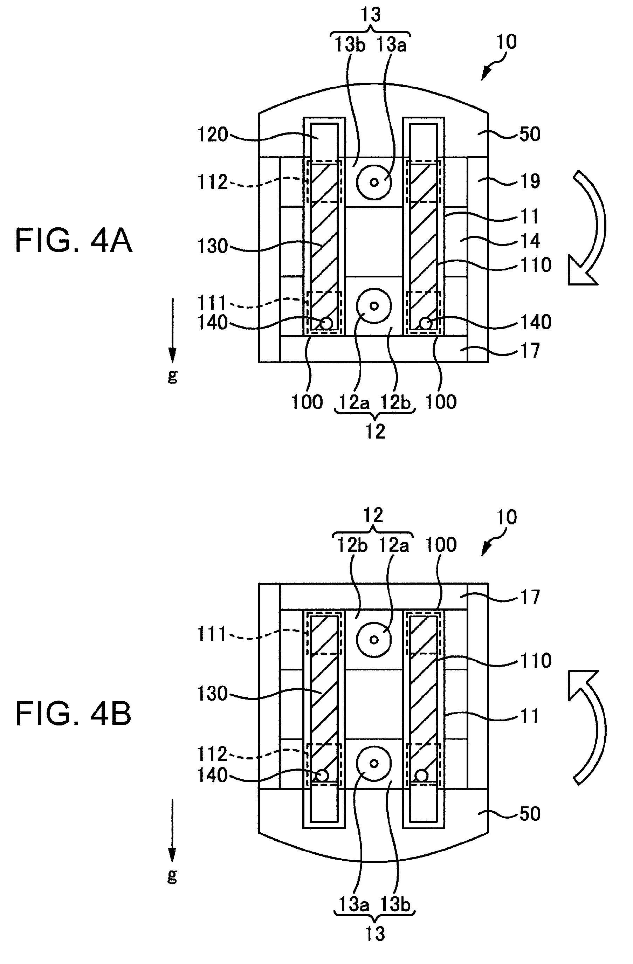

FIGS. 2A and 2B show one example of an up-and-down type PCR apparatus 1. FIG. 2A shows a state in which a lid 50 of the up-and-down type PCR apparatus 1 is closed, and FIG. 2B shows a state in which the lid 50 of the up-and-down type PCR apparatus 1 is opened and the nucleic acid amplification reaction tube 100 is fitted in a fitting section 11. FIG. 3 is an exploded perspective view of a main body 10 of the up-and-down type PCR apparatus 1 according to the embodiment. FIGS. 4A and 4B are cross- sectional views schematically showing the cross section taken along the line A-A of FIG. 2A of the main body 10 of the up-and-down type PCR apparatus 1 according to the embodiment.

This up-and-down type PCR apparatus 1 includes the main body 10 and a driving mechanism 20 as shown in FIG. 2A. As shown in FIG. 3, the main body 10 includes the fitting section 11, a first heating section 12, and a second heating section 13. A spacer 14 is provided between the first heating section 12 and the second heating section 13. In the main body 10 of this embodiment, the first heating section 12 is disposed on the bottom plate 17 side, and the second heating section 13 is disposed on the lid 50 side. In the main body 10 of this embodiment, the first heating section 12, the second heating section 13, and the spacer 14 are fixed by a flange 16, the bottom plate 17, and a fixing plate 19.

The fitting section 11 is configured such that the nucleic acid amplification reaction tube 100, which will be described later, is fitted therein. As shown in FIG. 2B and FIG. 3, the fitting section 11 of this embodiment has a slot structure in which the nucleic acid amplification reaction tube 100 is inserted and fitted, and is configured such that the nucleic acid amplification reaction tube 100 is inserted into a hole penetrating a first heat block 12b of the first heating section 12, the spacer 14, and a second heat block 13b of the second heating section 13. The number of the fitting sections 11 may be more than one, and in the example shown in FIG. 2B, twenty fitting sections 11 are provided for the main body 10.

This up-and-down type PCR apparatus 1 includes a structure in which the nucleic acid amplification reaction tube 100 is held at a predetermined position with respect to the first heating section 12 and the second heating section 13. More specifically, as shown in FIGS. 4A and 4B, in a flow channel 110 constituting the nucleic acid amplification reaction tube 100, which will be described later, a first region 111 can be heated by the first heating section 12 and a second region 112 can be heated by the second heating section 13. In this embodiment, a structure that defines the position of the nucleic acid amplification reaction tube 100 is the bottom plate 17, and as shown in FIG. 4A, by inserting the nucleic acid amplification reaction tube 100 to a position where the tube is in contact with the bottom plate 17, the nucleic acid amplification reaction tube 100 can be held at a predetermined position with respect to the first heating section 12 and the second heating section 13.

When the nucleic acid amplification reaction tube 100 is fitted in the fitting section 11, the first heating section 12 heats the first region 111 of the nucleic acid amplification reaction tube 100, which will be described later, to a first temperature. In the example shown in FIG. 4A, in the main body 10, the first heating section 12 is disposed at a position where the first region 111 of the nucleic acid amplification reaction tube 100 is heated.

The first heating section 12 may include a mechanism that generates heat and a member that transfers the generated heat to the nucleic acid amplification reaction tube 100. In the example shown in FIG. 3, the first heating section 12 includes a first heater 12a and a first heat block 12b. In this embodiment, the first heater 12a is a cartridge heater and is connected to an external power source (not shown) through a conductive wire 15. The first heater 12a is inserted into the first heat block 12b, and the first heat block 12b is heated by heat generated by the first heater 12a. The first heat block 12b is a member that transfers heat generated by the first heater 12a to the nucleic acid amplification reaction tube 100 . In this embodiment, the first heat block 12b is a block made of aluminum.

When the nucleic acid amplification reaction tube 100 is fitted in the fitting section 11, the second heating section 13 heats the second region 112 of the nucleic acid amplification reaction tube 100 to a second temperature different from the first temperature. In the example shown in FIG. 4A, in the main body 10, the second heating section 13 is disposed at a position where the second region 112 of the nucleic acid amplification reaction tube 100 is heated. As shown in FIG. 3, the second heating section 13 includes a second heater 13a and the second heat block 13b. The second heating section 13 is configured in the same manner as the first heating section 12 except that the region of the nucleic acid amplification reaction tube 100 to be heated and the heating temperature are different from those for the first heating section 12.

In this embodiment, the temperatures of the first heating section 12 and the second heating section 13 are controlled by a temperature sensor (not shown) and a control section (not shown), which will be described later. The temperatures of the first heating section 12 and the second heating section 13 are preferably set so that the nucleic acid amplification reaction tube 100 is heated to a desired temperature. In this embodiment, by controlling the first heating section 12 at the first temperature and the second heating section 13 at the second temperature, the first region 111 of the nucleic acid amplification reaction tube 100 can be heated to the first temperature, and the second region 112 can be heated to the second temperature. The temperature sensor in this embodiment is a thermocouple.

The driving mechanism 20 is a mechanism that controls the fitting section 11, the first heating section 12, and the second heating section 13, and by the driving mechanism, the arrangement of the first region and the second region is controlled. In this embodiment, the driving mechanism 20 includes a motor (not shown) and a drive shaft (not shown), and the drive shaft is connected to the flange 16 of the main body 10. The drive shaft in this embodiment is provided perpendicular to the longitudinal direction of the fitting section 11, and when the motor is activated, the main body 10 is rotated about the drive shaft as the axis of rotation.

The up-and-down type PCR apparatus 1 of this embodiment includes the control section (not shown). The control section controls at least one of the first temperature, the second temperature, a first period, a second period, and the cycle number of thermal cycles, which will be described later. In the case where the control section controls the first period or the second period, the control section controls the operation of the driving mechanism 20, thereby controlling the period in which the fitting section 11, the first heating section 12, and the second heating section 13 are held in a predetermined arrangement. In the embodiment of the invention, the control section controls the driving mechanism such that the nucleic acid amplification reaction mixture 140 is maintained in the first region at the first temperature for 4.5 seconds or less, preferably 4 seconds or less (a first period), and then, the nucleic acid amplification reaction mixture 140 is allowed to move into the second region at the second temperature and maintained in the second region for 18 seconds or less, preferably 12 seconds or less, more preferably 6 seconds or less (a second period). Here, the second temperature is lower than the first temperature. The control section may have mechanisms different from item to item to be controlled, or may control all items collectively. However, the control section in the up-and-down type PCR apparatus 1 of this embodiment is an electronic control system and controls all of the above-mentioned items. The control section of this embodiment includes a processor such as a CPU (not shown) and a storage device such as an ROM (Read Only Memory) or an RAM (Random Access Memory). In the storage device, various programs, data, etc. for controlling the above-mentioned respective items are stored. Further, the storage device has a work area for temporarily storing data in processing, processing results, etc. of various processes.

As shown in the example of FIG. 3 and FIG. 4A, in the main body 10 of this embodiment, the spacer 14 is provided between the first heating section 12 and the second heating section 13. The spacer 14 of this embodiment is a member that holds the first heating section 12 or the second heating section 13. In this embodiment, the spacer 14 is a heat insulating material, and in the example shown in FIG. 4A, the fitting section 11 penetrates the spacer 14.

The main body 10 of this embodiment includes the fixing plate 19. The fixing plate 19 is a member that holds the fitting section 11, the first heating section 12, and the second heating section 13. In the example shown in FIG. 2B and FIG. 3, two fixing plates 19 are fitted in the flanges 16, and the first heating section 12, the second heating section 13, and the bottom plate 17 are fixed by the fixing plates 19.

The up-and-down type PCR apparatus 1 of this embodiment includes the lid 50. In the example shown in FIG. 2A and FIG. 4A, the fitting section 11 is covered with the lid 50. The lid 50 may be fixed to the main body 10 by a fixing section 51. In this embodiment, the fixing section 51 is a magnet. As shown in the example of FIG. 2B and FIG. 3, a magnet is provided on a surface of the main body 10 which comes into contact with the lid 50. Although not shown in FIG. 2B and FIG. 3, a magnet is provided also for the lid 50 at a place where the magnet of the main body 10 comes into contact. When the fitting section 11 is covered with the lid 50, the lid 50 is fixed to the main body 10 by a magnetic force.

It is preferred that the fixing plate 19, the bottom plate 17, the lid 50, and the flange 16 are formed using a heat insulating material.

### (3) Thermal Cycling Treatment Using Up-and-down type PCR Apparatus

The nucleic acid amplification method according to the invention includes performing a cycle at predetermined times, the cycle including: maintaining a nucleic acid amplification reaction mixture in a first region at a first temperature of a liquid which is immiscible with the nucleic acid amplification reaction mixture for 4.5 seconds or less, preferably 4 seconds or less; allowing the nucleic acid amplification reaction mixture to move into a second region at a second temperature lower than the first temperature; and maintaining the nucleic acid amplification reaction mixture in the second region for 18 seconds or less, preferably 12 seconds or less, more preferably 6 seconds or less, wherein the concentration of magnesium ions in the nucleic acid amplification reaction mixture is 1.8 mM or more and 9.0 mM or less. Hereinafter, an example of an embodiment realizing this method will be described.

FIGS. 4A and 4B are cross-sectional views schematically showing the cross section taken along the line A-A of FIG. 2A of the up-and-down type PCR apparatus 1. FIGS. 4A and 4B show a state in which the nucleic acid amplification reaction tube 100 is fitted in the up-and-down type PCR apparatus 1. FIG. 4A shows a first arrangement and FIG. 4B shows a second arrangement. Hereinafter, a thermal cycling treatment using the up-and-down type PCR apparatus 1 according to the embodiment in the case of using the nucleic acid amplification reaction tube 100 will be described.

As shown in the example of FIG. 1, the nucleic acid amplification reaction tube 100 according to the embodiment includes a flow channel 110 and a sealing section 120. The flow channel 110 is filled with a reaction mixture 140 and a liquid 130 which has a specific gravity smaller than that of the reaction mixture 140 and is immiscible with the reaction mixture 140, and sealed with the sealing section 120.

The flow channel 110 is formed such that the reaction mixture 140 moves in close proximity to opposed inner walls. Here, the phrase "opposed inner walls" of the flow channel 110 refers to two regions of a wall surface of the flow channel 110 having an opposed positional relationship. The phrase "in close proximity to" refers to a state in which the distance between the reaction mixture 140 and the wall surface of the flow channel 110 is close, and includes a case where the reaction mixture 140 is in contact with the wall surface of the flow channel 110. Therefore, the phrase "the reaction mixture 140 moves in close proximity to opposed inner walls" refers to that "the reaction mixture 140 moves in a state of being close in distance to both of the two regions of a wall surface of the flow channel 110 having an opposed positional relationship", that is, the reaction mixture 140 moves along the opposed inner walls.

In the example shown in Fig. 1, the outer shape of the nucleic acid amplification reaction tube 100 is a cylindrical shape, and the flow channel 110 is formed in the direction of the center axis (the vertical direction in Fig. 1) therein. The shape of the flow channel 110 is a cylindrical shape having a circular cross section perpendicular to the longitudinal direction of the flow channel 110, that is, perpendicular to the direction in which the reaction mixture 140 moves in a region in the flow channel 110 (this cross section is defined as the "cross section" of the flow channel 110). Therefore, in the nucleic acid amplification reaction tube 100 of this embodiment, the opposed inner walls of the flow channel 110 are regions including two points on the wall surface of the flow channel 110 constituting the diameter of the cross section of the flow channel 110, and the reaction mixture 140 moves in the longitudinal direction of the flow channel 110 along the opposed inner walls.

The first region 111 of the nucleic acid amplification reaction tube 100 is a partial region of the flow channel 110 which is heated to the first temperature by the first heating section 12. The second region 112 is a partial region of the flow channel 110, which is different from the first region 111, and is heated to the second temperature by the second heating section 13. In the nucleic acid amplification reaction tube 100 of this embodiment, the first region 111 is a region including one end portion in the longitudinal direction of the flow channel 110, and the second region 112 is a region including the other end portion in the longitudinal direction of the flow channel 110. In the example shown in FIGS. 4A and 4B, a region surrounded by the dotted line including an end portion on the proximal side of the sealing section 120 of the flow channel 110 is the second region 112, and a region surrounded by the dotted line including an end portion on the distal side of the sealing section 120 is the first region 111.

As shown in FIG. 1, the flow channel 110 contains the liquid 130 and a liquid droplet of the reaction mixture 140. The liquid 130 and the reaction mixture 140 are prepared according to the description of (1) Nucleic Acid Amplification Reaction Vessel.

Hereinafter, with reference to FIGS. 4A and 4B, the thermal cycling treatment using the up-and-down type PCR apparatus 1 according to the embodiment will be described. In FIGS. 4A and 4B, the direction indicated by the arrow g (in the downward direction in the drawing) is the direction of the gravitational force. In this embodiment, a case where shuttle PCR (two-stage temperature PCR) is performed as an example of the thermal cycling treatment will be described. The respective steps described below show one example of the thermal cycling treatment, and according to need, the order of the steps may be changed, two or more steps may be performed continuously or concurrently, or a step may be added.

The shuttle PCR is a method of amplifying a nucleic acid in a reaction mixture by subjecting the reaction mixture to a two-stage temperature treatment at a high temperature and a low temperature repeatedly. In the treatment at a high temperature, denaturation of a double-stranded DNA occurs and in the treatment at a low temperature, annealing (a reaction in which a primer binds to a single-stranded DNA) and elongation (a reaction in which a complementary strand to the DNA is synthesized by using the primer as a starting point) occur.

In general, in shuttle PCR, the high temperature is a temperature between 80 °C and 100 °C and the low temperature is a temperature between 50°C and 70°C. The treatments at the respective temperatures are performed for a predetermined period, and a period in which the reaction mixture is maintained at a high temperature is generally shorter than a period in which the reaction mixture is maintained at a low temperature. The period of the treatment at a high temperature and the period of the treatment at a low temperature are not particularly limited, and may be arbitrarily set according to a nucleic acid to be amplified, the type of a primer, or the like. For example, the period of the treatment at a high temperature may be set to about 1 to 10 seconds, and the period of the treatment at a low temperature may be set to about 10 to 60 seconds, or a period longer than this range may be adopted depending on the condition of the reaction. In order to decrease the total period required for the nucleic acid amplification reaction, preferably, the period of the treatment at a high temperature is 4.5 seconds or less and the period of the treatment at a low temperature is 18 seconds or less, or the period of the treatment at a high temperature is 4 seconds or less and the period of the treatment at a low temperature is 6 seconds or less.

The appropriate period, temperature, and cycle number (the number of repetitions of the treatment at a high temperature and the treatment at a low temperature) vary depending on the type or amount of a reagent to be used, and therefore, it is preferred to determine an appropriate protocol in consideration of the type of a reagent or the amount of the reaction mixture 140 before performing the reaction.

First, the nucleic acid amplification reaction tube 100 is fitted in the fitting section 11. In this embodiment, after the reaction mixture 140 is introduced into the flow channel 110 previously filled with the liquid 130, the nucleic acid amplification reaction tube 100 is sealed with the sealing section 120, and then fitted in the fitting section 11. The introduction of the reaction mixture 140 can be performed using a micropipette, an ink-jet dispenser, or the like. In a state in which the nucleic acid amplification reaction tube 100 is fitted in the fitting section 11, the first heating section 12 is in contact with the nucleic acid amplification reaction tube 100 at a position including the first region 111 and the second heating section 13 is in contact with the nucleic acid amplification reaction tube 100 at a position including the second region 112.

Here, the arrangement of the fitting section 11, the first heating section 12, and the second heating section 13 is the first arrangement. As shown in FIG. 4A, in the first arrangement, the first region 111 of the nucleic acid amplification reaction tube 100 is located in a lowermost portion of the flow channel 110 in the direction of the gravitational force. In the first arrangement, the first region 111 is located in a lowermost portion of the flow channel 110 in the direction of the gravitational force, and therefore, the reaction mixture 140 having a specific gravity larger than that of the liquid 130 is located in the first region 111. In this embodiment, after the nucleic acid amplification reaction tube 100 is fitted in the fitting section 11, the fitting section 11 is covered with the lid 50, and then the up-and-down type PCR apparatus 1 is operated.

Subsequently, the nucleic acid amplification reaction tube 100 is heated by the first heating section 12 and the second heating section 13. The first heating section 12 and the second heating section 13 heat different regions of the nucleic acid amplification reaction tube 100 to different temperatures. That is, the first heating section 12 heats the first region 111 to the first temperature, and the second heating section 13 heats the second region 112 to the second temperature. According to this, a temperature gradient in which the temperature gradually changes between the first temperature and the second temperature is formed between the first region 111 and the second region 112 of the flow channel 110. Here, a temperature gradient in which the temperature decreases from the first region 111 to the second region 112 is formed. The thermal cycling treatment of this embodiment is shuttle PCR, and therefore, the first temperature is set to a temperature suitable for the denaturation of a double-stranded DNA, and the second temperature is set to a temperature suitable for annealing and elongation.

Since the arrangement of the fitting section 11, the first heating section 12, and the second heating section 13 is the first arrangement, when the nucleic acid amplification reaction tube 100 is heated, the nucleic acid amplification reaction mixture 140 is heated to the first temperature. When the first period has elapsed, the main body 10 is driven by the driving mechanism 20, and the arrangement of the fitting section 11, the first heating section 12, and the second heating section 13 is switched over from the first arrangement to the second arrangement. The second arrangement is an arrangement in which the second region 112 is located in a lowermost portion of the flow channel 110 in the direction of the gravitational force. In other words, the second region 112 is a region located in a lowermost portion of the flow channel 110 in the direction of the gravitational force when the fitting section 11, the first heating section 12, and the second heating section 13 are placed in a predetermined arrangement different from the first arrangement. In the up-and-down type PCR apparatus 1 of this embodiment, under the control of the control section, the driving mechanism 20 rotatively drives the main body 10. When the flanges 16 are rotatively driven by the motor by using the drive shaft as the axis of rotation, the fitting section 11, the first heating section 12, and the second heating section 13 which are fixed to the flanges 16 are rotated. Since the drive shaft is a shaft extending in the direction perpendicular to the longitudinal direction of the fitting section 11, when the drive shaft is rotated by the activation of the motor, the fitting section 11, the first heating section 12, and the second heating section 13 are rotated. In the example shown in FIGS. 4A and 4B, the main body 10 is rotated at 180°. By doing this, the arrangement of the fitting section 11, the first heating section 12, and the second heating section 13 is switched over from the first arrangement to the second arrangement.

Here, the positional relationship between the first region 111 and the second region 112 in the direction of the gravitational force is opposite to that of the first arrangement, and therefore, the reaction mixture 140 moves from the first region 111 to the second region 112 by the action of gravity. When the operation of the driving mechanism 20 is stopped after the arrangement of the fitting section 11, the first heating section 12, and the second heating section 13 has reached the second arrangement, the fitting section 11, the first heating section 12, and the second heating section 13 are held in the second arrangement. When the second period has elapsed in the second arrangement, the main body is rotated again. A nucleic acid amplification reaction is performed by rotation while switching over between the first arrangement and the second arrangement in this manner until completion of a predetermined number of cycles. An operation in which the first arrangement and the second arrangement are switched over once is defined as one cycle.

A period in which the nucleic acid amplification reaction mixture 140 moves from the first region to the second region can be arbitrarily set, but is preferably shorter. For example, the period may be 5 seconds or less, but is more preferably 2 seconds or less, and most preferably 1 second or less.

In this embodiment, as the PCR method, shuttle PCR is used, however, a PCR method (three-stage temperature PCR) in which the temperature is changed in thermal denaturation, annealing, and elongation reactions may be adopted. In this case, in addition to an annealing temperature, an elongation reaction temperature is set, and after the nucleic acid amplification reaction mixture is maintained at the annealing temperature, it may be maintained at the elongation reaction temperature for a predetermined period of time.

### (4) Cartridge

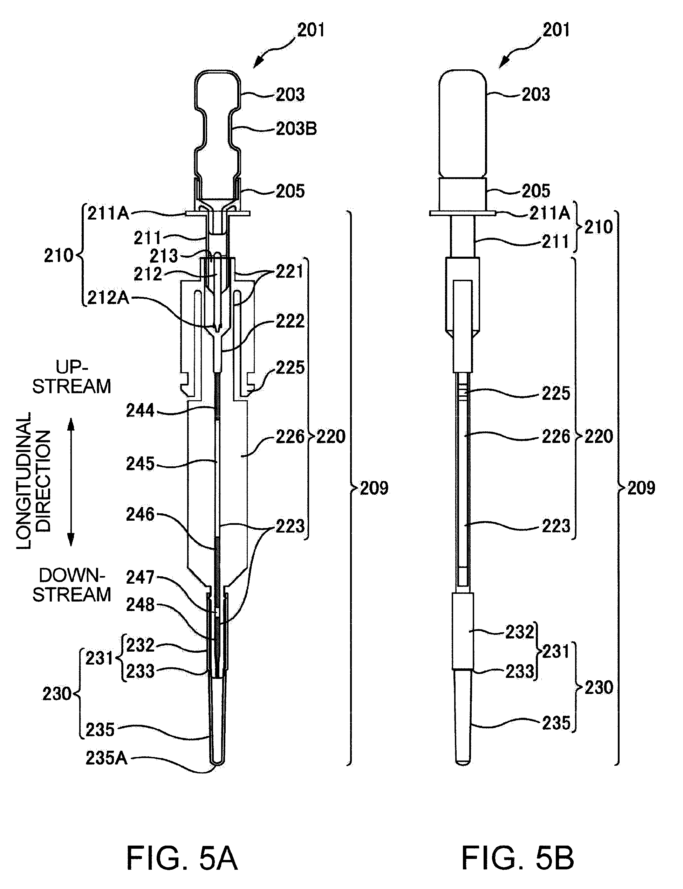

The nucleic acid amplification reaction vessel according to the invention may communicate with a cartridge 201 illustrated in FIGS. 5A and 5B. The cartridge shown in FIGS. 5A and 5B has the same configuration as described in JP-A-2014-176304. Here, the configuration and usage of this cartridge will be briefly described.

The cartridge 201 is composed of a tank 203 and a cartridge main body 209 including a plunger 210, a tube 220, and a nucleic acid amplification reaction vessel 230. In a kit constituting the cartridge 201, along with the tank 203 and the cartridge main body 209, an adapter 205 is prepared in advance. The cartridge 201 can be assembled by connecting the tank 203 and the cartridge main body 209 to each other through the adapter 205. It is also possible to constitute the cartridge 201 by directly attaching the tank 203 to the cartridge main body 209.

In the tank 203, a nucleic acid extraction treatment is performed, and a nucleic acid is bound to magnetic beads (not shown) . The tube 220 is internally provided with a first oil plug 244, a washing liquid plug 245, a second oil plug 246, an eluent plug 247, and a third oil plug 248 in the order from the upstream side shown in FIG. 5A. That is, an oil plug is disposed on both sides of a water-soluble plug (here, the washing liquid plug 245 or the eluent plug 247). Here, the "plug" refers to a specific liquid in the case where the liquid makes up one section in the tube. In other words, the liquids form layers, i.e. plugs, in the tube. An oil is phase-separated from other solutions (immiscible with other solutions), and therefore, the plug composed of an oil has a function of preventing the water-soluble plugs disposed on both sides thereof from being mixed with each other. The type of the oil is not particularly limited, but the oil is preferably the same oil as used for the above-mentioned liquid 130 . It is preferred that air bubbles or other liquids are not present in the plugs or between plugs, however, air bubbles or other liquids may be present as long as the magnetic beads can pass through the plugs.

The magnetic beads introduced into the tube 220 from the tank 203 move inside the tube by moving a magnet outside and along the tube 220, pass through the washing liquid plug 245, and reach the eluent plug 247. A nucleic acid bound to the magnetic beads is washed with the washing liquid in the washing liquid plug 245 and eluted in the eluent plug 247.

The washing liquid plug 245 may be composed of a plurality of plugs by being divided by oil plugs. In the case where the washing liquid plug 245 is composed of a plurality of plugs, liquids in the respective plugs may be either the same or different. The liquids in the other plugs are not particularly limited as long as there is at least one washing liquid plug among the plugs, however, it is preferred that all the plugs are composed of a washing liquid. The number of the divided plugs constituting the washing liquid plug 245 can be suitably set in consideration of, for example, the length of the tube 220, the object to be washed, etc. At this time, the washing liquid on the closest side to the eluent is preferably an acidic solution, and the other washing liquids preferably contain an alcohol.

The eluent of the eluent plug 247 refers to a liquid with which a nucleic acid adsorbed on a nucleic acid-binding solid-phase carrier is eluted in the liquid from the carrier and thereafter a reverse transcription reaction and a polymerase reaction are performed. Therefore, the eluent may be water or a buffer, or may be prepared in advance so that the eluent after elution of a nucleic acid becomes a buffer solution to be used directly in a reverse transcription reaction and a polymerase reaction. A salt for forming a buffer is not particularly limited as long as it does not inhibit the enzymatic reaction, but a salt such as Tris, HEPES, PIPES, or a phosphate is preferably used. Further, the eluent may contain a reverse transcriptase, dNTPs, and a primer (oligonucleotide) for the reverse transcriptase for performing a reverse transcription reaction. It is preferred that the eluent further contains BSA (bovine serum albumin) or gelatin as a preventive agent for reaction inhibition. A solvent is preferably water, and more preferably a solvent which contains substantially no organic solvents such as ethanol and isopropyl alcohol and chaotropic substances.

The concentration of dNTPs or a salt to be contained in the eluent may be set to a concentration suitable for the enzyme to be used in the end in consideration of the concentration of dNTPs or a salt in a lyophilized nucleic acid amplification reaction reagent, however, the concentration of dNTPs may be set to generally 10 to 1000 µM, preferably 100 to 500 µM, and the concentration of Cl⁻ may be set to generally 1 to 2000 mM, preferably 200 to 700 mM. The total ion concentration is not particularly limited, but may be higher than 50 mM, and is preferably higher than 100 mM, more preferably higher than 120 mM, further more preferably higher than 150 mM, still further more preferably higher than 200 mM. The upper limit thereof is preferably 500 mM or less, more preferably 300 mM or less, further more preferably 200 mM or less. Each oligonucleotide for the primer is used at 0.1 to 10 µM, preferably at 0.1 to 1 µM. If the concentration of BSA or gelatin is 1 mg/mL or less, the preventive effect on reaction inhibition is small, and if the concentration thereof is 10 mg/mL or more, it may inhibit the reverse transcription reaction or the subsequent enzymatic reaction, and therefore, the concentration thereof is preferably from 1 to 10 mg/mL. In the case where gelatin is used, the gelatin may be derived from, for example, cattle skin, pig skin, or cattle bone, but the origin thereof is not particularly limited thereto. If the gelatin is sparsely soluble, it may be dissolved by heating.

In the nucleic acid amplification reaction vessel 230 communicating with the tube 220, the above-mentioned thermal cycling treatment is performed. The nucleic acid amplification reaction vessel 230 is filled with a liquid which is the same as the above-mentioned liquid 130 and is immiscible with a nucleic acid amplification reaction mixture. When the eluent plug 247 is pushed out into the nucleic acid amplification reaction vessel 230 from the tube 220, it is formed into a liquid droplet, and the eluent 247 formed into a liquid droplet precipitates. The eluent 247 precipitating in the nucleic acid amplification reaction vessel 230 is mixed with a nucleic acid amplification reaction mixture containing a nucleic acid amplification reaction reagent present in the nucleic acid amplification reaction vessel 230. The nucleic acid amplification reaction vessel 230 may contain a lyophilized nucleic acid amplification reaction reagent, and the lyophilized reagent may be dissolved in the eluent 247 to form the nucleic acid amplification reaction mixture.

As another embodiment, the tube 220 may include, in addition to the first oil plug 244, the washing liquid plug 245, the second oil plug 246, the eluent plug 247, and the third oil plug 248, a nucleic acid amplification reaction mixture plug and a fourth oil plug provided downstream thereof. In this case, a mixed liquid obtained by mixing the eluent and the nucleic acid amplification reaction mixture is pushed out into the nucleic acid amplification reaction vessel.

In the nucleic acid amplification reaction vessel 230, a first region heated by the above-mentioned heating section to a first temperature and a second region heated by the above-mentioned heating section to a second temperature lower than the first temperature are formed, and by repeatedly turning upside down the entire cartridge 101 along with the heating sections, the nucleic acid amplification reaction mixture in the form of a liquid droplet moves between the first region and the second region to effect a thermal cycling treatment, whereby a nucleic acid is amplified.

The configuration of the apparatus for performing the nucleic acid amplification reaction by rotating the nucleic acid amplification reaction vessel communicating with the cartridge as described above is not particularly limited. For example, the thermal cycling treatment according to the invention can be performed by rotating the nucleic acid amplification reaction vessel according to the configuration of the apparatus described in the above-mentioned JP-A-2014-176304.

In the case where the cartridge as illustrated above is used, the concentration of magnesium ions in the mixed liquid of the eluent and the nucleic acid amplification reaction mixture may be set to 1.8 mM or more and 9.0 mM or less, preferably 2.0 mM or more and 7.5 mM or less.

According to still another embodiment, the eluent plug 247 contains also the above-mentioned nucleic acid amplification reaction reagent, and the above-mentioned thermal cycling treatment may be performed for the eluent 247 in the form of a liquid droplet pushed out into the nucleic acid amplification reaction vessel 230. That is, in this embodiment, it is not necessary that the nucleic acid amplification reaction vessel should contain the nucleic acid amplification reaction reagent, and the concentration of magnesium ions in the eluent may be set to 1.8 mM or more and 9.0 mM or less, preferably 2.0 mM or more and 7.5 mM or less.

The cartridges as illustrated herein can be used for extracting and/or purifying nucleic acids.

### Examples

Hereinafter, the invention will be described in more detail by showing Examples, however, the invention is not limited thereto.

### Example 1: Relationship between Concentration of Magnesium Ions and Nucleic Acid Amplification Reaction Efficiency in PCR

Apparatus according to Related Art and PCR Apparatus according to the Invention

### (1) In the Case of using PCR Apparatus according to Related Art (Comparative Example)

In Comparative Example, by using StepOnePlus of Life Technologies, Inc. as the PCR apparatus, a nucleic acid amplification reaction (PCR) was performed by using a plasmid DNA of a type A influenza virus (hereinafter also referred to as "InfA") as the template. Here, a shuttle PCR method in which the annealing temperature and the elongation temperature are the same was used.

A reaction mixture in which the concentration of magnesium ions in the nucleic acid amplification reaction mixture was 1.5 mM was prepared and named "reaction mixture 1", and a reaction mixture in which the concentration of magnesium ions in the nucleic acid amplification reaction mixture was 5.0 mM was prepared and named "reaction mixture 2".

The compositions of the reaction mixtures 1 and 2 are as follows. In the nucleic acid amplification reaction, 10 µL of the nucleic acid amplification reaction mixture was used, and as the liquid amount of each component described below, a liquid amount per 10 µL of the reaction mixture is shown.

### (Reaction mixture 1)

| | |
|---|---|
| Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfA plasmid DNA | 1.0 µL |
| Water | 3.9 µL |
| | Total: 10.0 µL |

| | |
|---|---|
| * Composition of 5x buffer: MgCl₂: 7.5 mM, KCl: 125 mM, Tris-HCl (pH 9.0): 250 mM * Final concentration in reaction mixture: MgCl₂ : 1.5 mM, KCl: 25 mM, Tris-HCl (pH 9.0): 50 mM | |

### (Reaction mixture 2)

| | |
|---|---|
| Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfA plasmid DNA | 1.0 µL |
| Water | 3.9 µL |
| | Total: 10.0 µL |

| | |
|---|---|
| * Composition of 5x buffer: MgCl₂: 25 mM, KCl: 125 mM, Tris-HCl (pH 9.0): 250 mM Final concentration: MgCl₂: 5 mM, KCl: 25 mM, Tris-HCl (pH9.0) : 50 mM | |

Further, the sequences of the primers and the probe are as follows.

### (Sequences of Primers)

InfA forward primer: 5'- GACCRATCCTGTCACCTCTGAC -3' (SEQ ID NO: 1)
InfA reverse primer: 5'- AGGGCATTYTGGACAAAKCGTCTA -3 ' (SEQ ID NO: 2)

### (Sequence of Probe)

InfA TaqMan probe: 5 ' FAM- CACAAATCCTAAAATTCCCT-BHQ1 - 3' (SEQ ID NO: 3)

The conditions for PCR are as follows.

### Conditions for hot start: 98°C for 2 min

Conditions for thermal cycle: thermal denaturation: 98°C for 15 sec, annealing and elongation: 55°C for 30 sec (50 cycles)

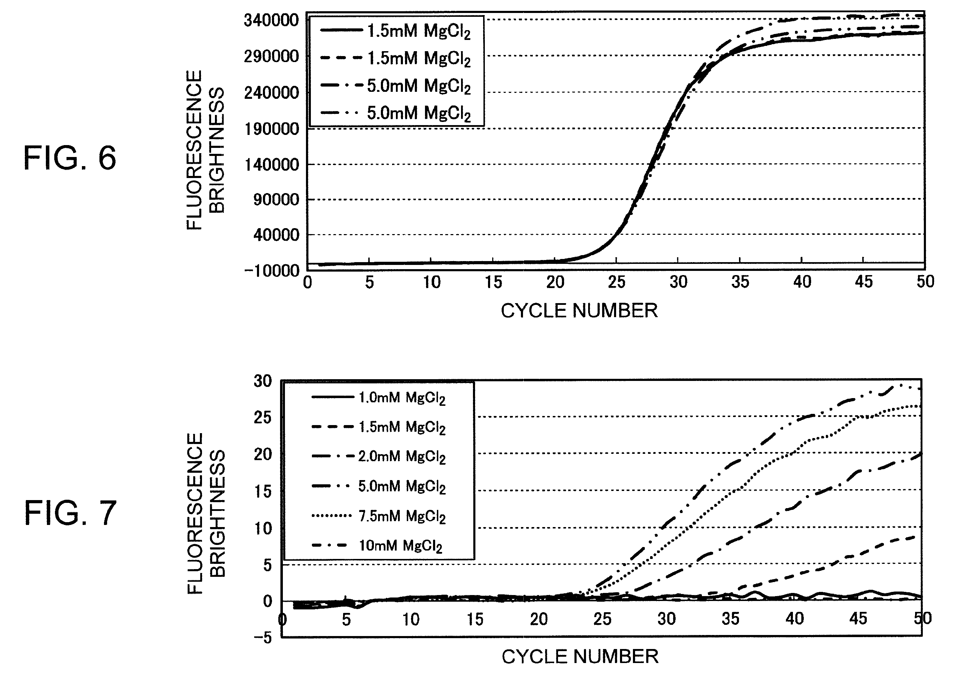

For each reaction mixture, PCR was performed twice. The results are shown in FIG. 6. A higher fluorescence brightness represented by the vertical axis indicates that a larger amount of a nucleic acid amplification reaction product is obtained.

As seen from FIG. 6, in the PCR apparatus of Comparative Example, the nucleic acid amplification reaction efficiency was equivalent in both cases where the concentration of magnesium ions in the reaction mixture was 1. 5 mM and where it was 5.0 mM. Here, with respect to the determination of high or low reaction efficiency, a case where the fluorescence brightness for the cycle number is high, the reaction efficiency is determined to be high, and a case where the fluorescence brightness for the cycle number is low, the reaction efficiency is determined to be low.

### (2) In the Case of using up-and-down type PCR Apparatus according to the Invention (Example)

The above-mentioned up-and-down type PCR apparatus was used in place of the PCR apparatus used in the above Comparative Example, 6 types of reaction mixtures in which the concentration of magnesium ions in the nucleic acid amplification reaction mixture was 1.0 mM, 1.5 mM, 2.0 mM, 5.0 mM, 7.5 mM, or 10.0 mM were prepared, and PCR was performed by using the above-mentioned InfA plasmid as the template and also using the above-mentioned primers and probe.

The compositions of the reaction mixtures are as follows.

| | |
|---|---|
| Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfA plasmid DNA | 1.0 µL |
| Water | 3.9 µL |
| | Total: 10.0 µL |

| | |
|---|---|
| * Composition of 5x buffer: MgCl₂: 5.0 mM, 7.5 mM, 10 mM, 25 mM, 37.5 mM, or 50.0 mM, KCl: 125 mM, Tris-HCl (pH 9.0): 250 mM * Final concentration: MgCl₂: 1.0 mM, 1.5 mM, 2.0 mM, 5.0 mM, 7.5 mM, or 10.0 mM, KCl: 25 mM, Tris-HCl (pH 9.0): 50 mM | |

The conditions for PCR are as follows.

### Conditions for hot start: 98°C for 10 sec Conditions for thermal cycle: 98°C for 3 sec and 60°C for 6 sec (50 cycles)

The results are shown in FIG. 7. The reaction efficiency was particularly high when the concentration of magnesium ions was 2.0 mM, 5.0 mM, and 7.5 mM. On the other hand, amplification was almost not observed when the concentration of magnesium ions was 1.0 mM and 10.0 mM.

That is, in the case where the PCR reaction time is short, there is a concentration range in which the reaction efficiency is improved as compared with the case where the concentration of magnesium ions is 1.5 mM within a range in which the concentration of magnesium ions is higher than 1.5 mM having been determined to be a recommended concentration.

### Example 2: Relationship among Concentration of Magnesium Ions, Concentration of Potassium Ions, and Nucleic Acid Amplification Reaction Efficiency in PCR Apparatus according to the Invention

### (1) Effect of Concentration of Potassium Ions when Concentration of Magnesium Ions is 1.5 mM

A nucleic acid amplification reaction was performed in the same manner as in Example 1 except that the concentration of magnesium ions in the nucleic acid amplification reaction mixture in Example 1 was set to 1.5 mM, the concentration of KCl in the nucleic acid amplification reaction mixture was set to 0 mM, 25 mM, or 65 mM, and 1.6 µL of the reaction mixture was used in the nucleic acid amplification reaction.

The compositions of the respective nucleic acid amplification reaction mixtures are as follows.

| | |
|---|---|
| Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfA plasmid DNA | 1.0 µL |
| Water | 3.9 µL |
| | Total: 10.0 µL |

| | |
|---|---|
| * Composition of 5x buffer: KCl: 0 mM, 125 mM, or 325 mM, MgCl₂: 7.5 mM, Tris-HCl (pH 9.0): 250 mM Final concentration: KCl: 0 mM, 25 mM, or 65 mM, MgCl₂: 1.5 mM, Tris-HCl (pH 9.0): 50 mM | |

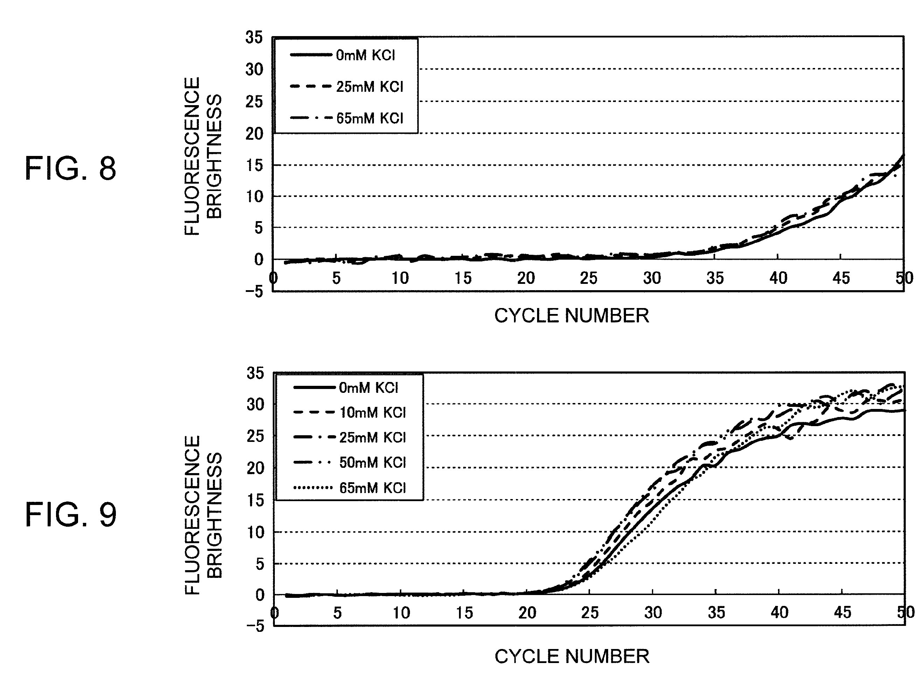

The results are shown in FIG. 8. The concentration of KCl did not affect the reaction efficiency.

### (2) Effect of Concentration of Potassium Ions when Concentration of Magnesium Ions is 5.0 mM

A nucleic acid amplification reaction was performed in the same manner as in Example 1 except that the concentration of magnesium ions in the nucleic acid amplification reaction mixture in Example 1 was set to 5.0 mM, and the concentration of KCl in the nucleic acid amplification reaction mixture was set to 0 mM, 10 mM, 25 mM, 50 mM, or 65 mM.

The compositions of the respective nucleic acid amplification reaction mixtures are as follows.

| | |
|---|---|
| Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfA plasmid DNA | 1.0 µL |
| Water | 3.9 µL |
| | Total: 10.0 µL |

| | |
|---|---|
| * Composition of 5x buffer: KCl: 0 mM, 50 mM, 125 mM, 250 mM, or 325 mM, MgCl₂: 25 mM, Tris-HCl (pH 9.0): 250 mM Final concentration: KCl: 0 mM, 10 mM, 25 mM, 50 mM, or 65 mM, MgCl₂: 5.0 mM, Tris-HCl (pH 9.0): 50 mM | |

The results are shown in FIG. 9. The concentration of KCl did not affect the reaction efficiency also in the case where the concentration of magnesium ions was 5.0 mM. Further, regardless of the concentration of KCl, the reaction efficiency was higher in the case where the concentration of magnesium ions was 5.0 mM than in the case where the concentration of magnesium ions was 1.5 mM.

### Example 3: Relationship between Concentration of Magnesium Ions and Nucleic Acid Amplification Reaction Efficiency in PCR Apparatus according to the Invention

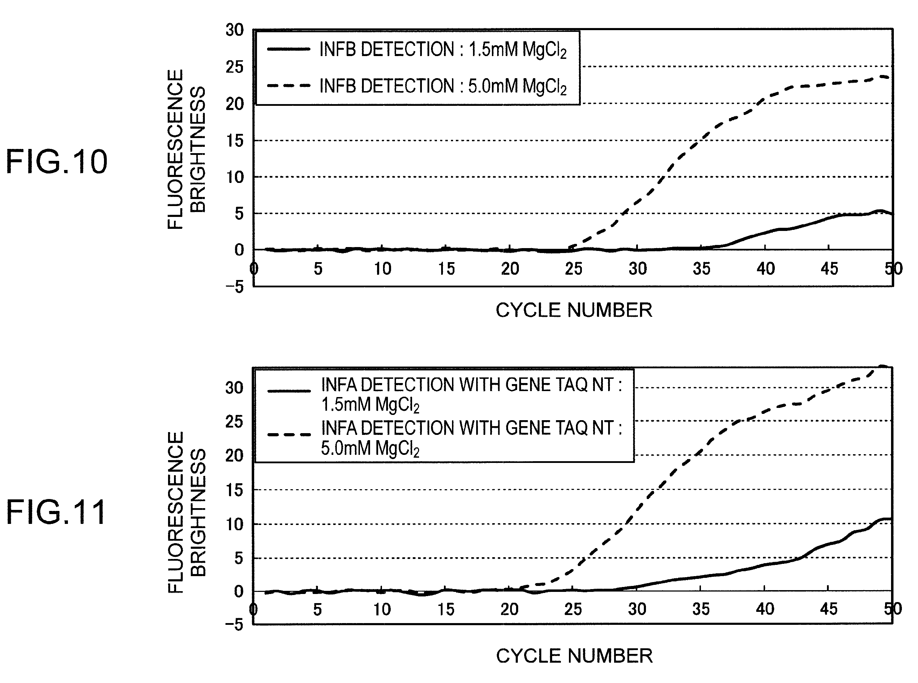

PCR was performed in the same manner as in Example 1 except that the template in Example 1 (2) was changed to a plasmid of InfB (a type B influenza virus), and the concentration of magnesium ions was set to 1.5 mM or 5.0 mM. The results are shown in FIG. 10.

As seen from FIG. 10, also in the case where the plasmid of InfB was used as the template, the same results as in the case where the plasmid of InfA was used as the template, that is, the results that the reaction efficiency was higher in the case where the concentration of magnesium ions was 5.0 mM than in the case where the concentration of magnesium ions was 1.5 mM were obtained. In other words, according to the invention, the reaction efficiency can be increased regardless of the type of a template in a nucleic acid amplification reaction.

### Example 4: In the Case of Changing Polymerase

RT-PCR was performed in the same manner as in Example 1 except that the polymerase in Example 1 was changed to Gene Taq NT, and the concentration of magnesium ions in the nucleic acid amplification reaction mixture was set to 1.5 mM or 5.0 mM. The results are shown in FIG. 11.

As seen from FIG. 11, also in the case where the Gene Taq NT (Nippon Gene Co. , Ltd.) was used as the DNA polymerase, the same results as in the case where Platinum Taq was used, that is, the results that the reaction efficiency was higher in the case where the concentration of magnesium ions was 5.0 mM than in the case where the concentration of magnesium ions was 1.5 mM were obtained. In other words, according to the invention, the reaction efficiency can be increased regardless of the type of DNA polymerase.

### Example 5: Presence or Absence of Non-specific Amplification Reaction in Single PCR

An RT-PCR reaction was performed by using a reaction mixture having the following composition in an amount of 10 µL in the above-mentioned PCR apparatus of Comparative Example, and in an amount of 1.6 µL in the above-mentioned up-and-down type PCR apparatus of Example.

### (Reaction mixture 1)

| | |
|---|---|
| SuperScript 3 + Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfA RNA | 1.0 µL |
| Water | 3.9 µL |
| | Total: 10.0 µL |

| | |
|---|---|
| * Composition of 5x buffer: MgCl₂: 25 mM, Tris-HCl (pH 9.0): 250 mM, KCl: 125 mM Final concentration: MgCl₂: 5 mM, Tris-HCl (pH 9.0): 50 mM, KCl: 25 mM | |

### (Reaction mixture 2)

| | |
|---|---|
| SuperScript 3 + Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfA RNA | 1.0 µL |
| Human total RNA | 1.0 µL |
| Water | 2.9 µL |
| | Total: 10.0 µL |

| | |
|---|---|
| * Composition of 5x buffer: MgCl₂: 25 mM, Tris-HCl (pH 9.0): 250 mM, KCl: 125 mM Final concentration: MgCl₂: 5 mM, Tris-HCl (pH 9.0): 50 mM, KCl: 25 mM | |

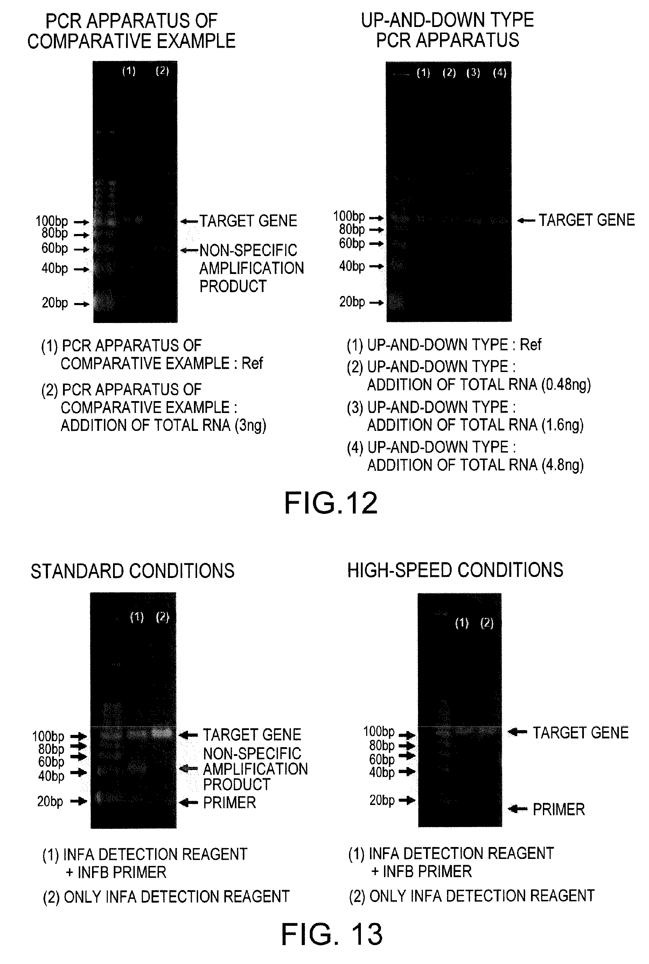

A difference between the reaction mixture 1 and the reaction mixture 2 is only whether human total RNA is contained or not. The amount of human total RNA is as described in the explanation of lanes (2), (3), and (4) in FIG. 12. Incidentally, as the sequences of the primers and the probe, the same ones as used in Example 1 were used.

The conditions for the RT-PCR reaction are as follows.

### (PCR Apparatus of Comparative Example)

Reverse transcription (RT) reaction: 50°C for 30 min Reverse transcriptase inactivation reaction: 98°C for 2 min Thermal cycle: 50 cycles were performed under the following conditions: 98°C for 15 sec and 55°C for 30 sec

### (Up-and-down type PCR Apparatus of Example)

Reverse transcription (RT) reaction: 50°C for 60 sec Reverse transcriptase inactivation reaction: 98°C for 10 sec Thermal cycle: 50 cycles were performed under the following conditions: 98°C for 4 sec and 60°C for 6 sec

In this test, the nucleic acid to be amplified was the RNA of InfA and the reaction mixture 2 contained human total RNA, and therefore, non-specific amplification was likely to occur, and the results of electrophoresis of the amplification products obtained under such conditions are shown in FIG. 12.

In FIG. 12, the lanes (1) of Comparative Example and Example are lanes of amplification products in the reaction mixture 1, that is, the reaction mixture which did not contain human total RNA, and the lanes (2), (3), and (4) are lanes of amplification products in the reaction mixture 2, that is, the reaction mixture which contained human total RNA.

First, when comparing the lanes (1) of Example and Comparative Example, in Comparative Example, a non-specific amplification product was produced considerably, however, in Example, the amplification of the target DNA mainly occurred, and almost no non-specific amplification product was produced.

Further, in the lane (2) of Comparative Example, non-specific amplification occurred to such an extent that the amplification of the target DNA could not be detected. On the other hand, in all of the lanes (2) to (4) of Example, the same results as in the case of the reaction mixture 1 which did not contain human total RNA were obtained, and even in the lane (4) in which PCR was performed using the reaction mixture 2 containing a larger amount of human total RNA than in Comparative Example, almost no non-specific amplification could be observed.

### Example 6: Presence or Absence of Non-specific Amplification Reaction in Multiplex PCR

A PCR reaction was performed in the up-and-down type PCR apparatus by taking out a 1.6 µL portion from each of the reaction mixtures 1 and 2 having the following composition.

### (Reaction mixture 1)

| | |
|---|---|
| Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfB forward primer (20 µM) | 0.8 µL |
| InfB reverse primer (20 µM) | 0.8 µL |
| InfA Plasmid DNA | 1.0 µL |
| Water | 2.3 µL |
| | Total: 10.0 µL |

| | |
|---|---|
| * Composition of 5x buffer: MgCl₂: 25 mM, Tris-HCl (pH 9.0) : 250 mM, KCl: 125 mM Final concentration: MgCl₂: 5 mM, Tris-HCl (pH 9.0): 50 mM, KCl: 25 mM | |

### (Reaction mixture 2)

| | |
|---|---|
| Platinum Taq | 0.4 µL |
| 5x buffer (*) | 2.0 µL |
| dNTPs (10 mM) | 0.5 µL |
| InfA forward primer (20 µM) | 0.8 µL |
| InfA reverse primer (20 µM) | 0.8 µL |
| InfA TaqMan probe (10 µM) | 0.6 µL |
| InfA Plasmid DNA | 1.0 µL |
| Water | 3.9 µL |
| | Total: 10.0 µL |

| | |
|---|---|
| * Composition of 5x buffer: MgCl₂: 25 mM, Tris-HCl (pH 9.0): 250 mM, KCl: 125 mM Final concentration: MgCl₂: 5 mM, Tris-HCl (pH 9.0): 50 mM, KCl: 25 mM | |

In the reaction mixture 1, a plasmid DNA of InfA was contained as the template and also a primer pair for amplifying InfA was contained, and in addition thereto, a primer pair which binds to a plasmid DNA of InfB was further contained. On the other hand, the reaction mixture 2 had the same composition as that of the reaction mixture 1 except that the primer pair for InfB was not contained.

As the sequences of the primers and the probes, the same ones as used in Example 1 were used for InfA, and the following sequences were used for InfB.

### (InfB primer pair)

InfB forward primer: 5'- TCCTCAACTCACTCTTCGAGCG -3' (SEQ ID NO: 4)
InfB reverse primer: 5' - CGGTGCTCTTGACCAAATTGG - 3' (SEQ ID NO: 5)

The conditions for the reaction are as follows.

### Hot start reaction: 98°C for 10 sec

Thermal cycle: 50 cycles were performed under the following conditions.
High-speed conditions (Example) : 98°C for 4 sec and 60°C for 6 sec
Standard conditions (Comparative Example): 98°C for 5 sec and 60°C for 20 sec

The results of electrophoresis of the mixture containing amplification products are shown in FIG. 13.

In FIG. 13, the lanes (1) of Comparative Example and Example are lanes in which the reaction mixture 1 containing the primer pair for InfB was loaded, and the lanes (2) are lanes in which the reaction mixture 2 containing no primer pair for InfB was loaded.

As seen from FIG. 13, in Comparative Example, a non-specific amplification product was observed in the lane (1), however, in the lane (1) of Example, mainly a specific amplification product could be confirmed. In this manner, as the PCR reaction time is shorter, non-specific amplification is less likely to occur.

## Claims

1. A nucleic acid amplification reaction apparatus, comprising:
a fitting section capable of fitting a nucleic acid amplification reaction vessel filled with a nucleic acid amplification reaction mixture and a liquid which has a specific gravity different from that of the nucleic acid amplification reaction mixture and is immiscible with the nucleic acid amplification reaction mixture;
a first heating section which heats a first region of the nucleic acid amplification reaction vessel to a first temperature;
a second heating section which heats a second region of the nucleic acid amplification reaction vessel to a second temperature; and
a driving mechanism which switches over between a first arrangement in which the first region is located lower than the second region in the direction of the gravitational force and a second arrangement in which the second region is located lower than the first region in the direction of the gravitational force, wherein
the concentration of magnesium ions in the nucleic acid amplification reaction mixture is 1.8 mM or more and 9.0 mM or less.

2. The nucleic acid amplification reaction apparatus according to claim 1, wherein the concentration of magnesium ions in the nucleic acid amplification reaction mixture is 2.0 mM or more and 7.5 mM or less.

3. The nucleic acid amplification reaction apparatus according to claim 1 or 2, wherein
the apparatus further comprises a control section which controls the driving mechanism, and
a period in which the control section maintains the first arrangement is 4.5 seconds or less, and a period in which the control section maintains the second arrangement is 18 seconds or less.

4. The nucleic acid amplification reaction apparatus according to claim 3, wherein a period in which the control section maintains the first arrangement is 4 seconds or less.

5. The nucleic acid amplification reaction apparatus according to claim 3, wherein a period in which the control section maintains the second arrangement is 6 seconds or less.

6. The nucleic acid amplification reaction apparatus according to any one of claims 1 to 5, wherein the liquid has a specific gravity smaller than that of the nucleic acid amplification reaction mixture.

7. The nucleic acid amplification reaction apparatus according to any one of claims 1 to 5, wherein the liquid has a specific gravity larger than that of the nucleic acid amplification reaction mixture.

8. A nucleic acid amplification reaction cartridge, comprising a tube, a nucleic acid amplification reaction vessel, and a plunger,
the tube being internally provided, in the following order, with
a first plug composed of a first oil,
a second plug composed of a washing liquid, which is immiscible with an oil, and with which a nucleic acid-binding solid-phase carrier having a nucleic acid bound thereto can be washed,
a third plug composed of a second oil,
a fourth plug composed of an eluent, which is immiscible with an oil, and with which the nucleic acid can be eluted from the nucleic acid-binding solid-phase carrier having the nucleic acid bound thereto,
a fifth plug composed of a third oil,
a sixth plug composed of a nucleic acid amplification reaction mixture, which is immiscible with an oil, and with which a nucleic acid amplification reaction can be performed, and
a seventh plug composed of a fourth oil,
the nucleic acid amplification reaction vessel communicating with the tube on the seventh plug side and containing an oil, and
the plunger being attached to an opening section of the tube on the first plug side so as to be suitable for pushing out the liquid from the tube on the seventh plug side to the nucleic acid amplification reaction vessel, wherein
the concentration of magnesium ions in a mixed liquid of the eluent and the nucleic acid amplification reaction mixture is 1.8 mM or more and 9.0 mM or less.

9. A nucleic acid amplification reaction cartridge, comprising a tube, a nucleic acid amplification reaction vessel, and a plunger,
the tube being internally provided, in the following order, with
a first plug composed of a first oil,
a second plug composed of a washing liquid, which is immiscible with an oil, and with which a nucleic acid-binding solid-phase carrier having a nucleic acid bound thereto can be washed,
a third plug composed of a second oil,
a fourth plug composed of an eluent, which is immiscible with an oil, and with which the nucleic acid can be eluted from the nucleic acid-binding solid-phase carrier having the nucleic acid bound thereto, and
a fifth plug composed of a third oil,
the nucleic acid amplification reaction vessel communicating with the tube on the fifth plug side and containing an oil, and
the plunger being attached to an opening section of the tube on the first plug side so as to be suitable for pushing out the liquid from the tube on the fifth plug side to the nucleic acid amplification reaction vessel, wherein
the nucleic acid amplification reaction vessel contains a liquid droplet composed of a nucleic acid amplification reaction mixture, which is immiscible with an oil, and with which a nucleic acid amplification reaction can be performed, and
the concentration of magnesium ions in a mixed liquid of the eluent and the nucleic acid amplification reaction mixture is 1.8 mM or more and 9.0 mM or less.

10. A nucleic acid amplification reaction cartridge, comprising a tube, a nucleic acid amplification reaction vessel, and a plunger,
the tube being internally provided, in the following order, with
a first plug composed of a first oil,
a second plug composed of a washing liquid, which is immiscible with an oil, and with which a nucleic acid-binding solid-phase carrier having a nucleic acid bound thereto can be washed,
a third plug composed of a second oil,
a fourth plug composed of an eluent, which is immiscible with an oil, and with which the nucleic acid can be eluted from the nucleic acid-binding solid-phase carrier having the nucleic acid bound thereto, and
a fifth plug composed of a third oil,
the nucleic acid amplification reaction vessel communicating with the tube on the fifth plug side and containing an oil, and
the plunger being attached to an opening section of the tube on the first plug side so as to be suitable for pushing out the liquid from the tube on the fifth plug side to the nucleic acid amplification reaction vessel, wherein
the concentration of magnesium ions in the eluent is 1.8 mM or more and 9.0 mM or less.

11. The nucleic acid amplification reaction cartridge according to claim 10, wherein the eluent contains the nucleic acid amplification reaction reagent.

12. A nucleic acid amplification method, comprising:
heating a first region of a nucleic acid amplification reaction vessel filled with a nucleic acid amplification reaction mixture and a liquid which has a specific gravity different from that of the nucleic acid amplification reaction mixture and is immiscible with the nucleic acid amplification reaction mixture to a first temperature;
heating a second region of the nucleic acid amplification reaction vessel to a second temperature lower than the first temperature;
maintaining a first arrangement in which the first region is located lower than the second region in the direction of the gravitational force for a first period so as to allow the nucleic acid amplification reaction mixture to move into the first region;
maintaining a second arrangement in which the second region is located lower than the first region in the direction of the gravitational force for a second period so as to allow the nucleic acid amplification reaction mixture to move into the second region, wherein
the concentration of magnesium ions in the nucleic acid amplification reaction mixture is 1.8 mM or more and 9.0 mM or less.

13. The nucleic acid amplification method according to claim 12, wherein
the first period is 4.5 seconds or less, and
the second period is 18 seconds or less.

14. The nucleic acid amplification method according to claim 12 or 13, wherein the liquid has a specific gravity smaller or larger than that of the nucleic acid amplification reaction mixture.

15. A nucleic acid amplification method, comprising performing a cycle at predetermined times, the cycle including:
maintaining a nucleic acid amplification reaction mixture in a first region at a first temperature of a liquid which is immiscible with the nucleic acid amplification reaction mixture for 4.5 seconds or less;
allowing the nucleic acid amplification reaction mixture to move into a second region at a second temperature lower than the first temperature; and
maintaining the nucleic acid amplification reaction mixture in the second region for 18 seconds or less, wherein
the concentration of magnesium ions in the nucleic acid amplification reaction mixture is 1.8 mM or more and 9.0 mM or less.


SEQUENCE LISTING

<110> Seiko Epson Corporation

<120> Nucleic acid amplification device and method for
amplifying nucleic acid

<130> J018276201

<160> 5

<170> PatentIn version 3.5

<210> 1
<211> 22
<212> DNA
<213> Artificial Sequence

<220>
<223> PCR forward primer

<400> 1
gaccratcct gtcacctctg ac                                          22

<210> 2

<211> 24

<212> DNA

<213> Artificial Sequence

<220>

<223> PCR reverse primer

<400> 2

agggcattyt ggacaaakcg tcta                                              24

<210> 3

<211> 20

<212> DNA

<213> Artificial Sequence

<220>

<223> PCR probe

<400> 3

cacaaatcct aaaattccct                                                   20

<210> 4

<211> 22

<212> DNA

<213> Artificial Sequence

<220>

<223> PCR forward primer

<400> 4

tcctcaactc actcttcgag cg                                              22

<210> 5

<211> 21

<212> DNA

<213> Artificial Sequence

<220>

<223> PCR reverse primer

<400> 5

cggtgctctt gaccaaattg g                                               21

SEQUENCE LISTING

<110> Seiko Epson Corporation

<120> Nucleic acid amplification device and method for amplifying nucleic
acid

<130> J018276201

<160> 5

<170> PatentIn version 3.5

<210> 1
<211> 22
<212> DNA
<213> Artificial Sequence

<220>
<223> PCR forward primer

<400> 1
gaccratcct gtcacctctg ac                                              22


<210> 2
<211> 24
<212> DNA
<213> Artificial Sequence

<220>
<223> PCR reverse primer

<400> 2
agggcattyt ggacaaakcg tcta                                            24


<210> 3
<211> 20
<212> DNA
<213> Artificial Sequence

<220>
<223> PCR probe

<400> 3
cacaaatcct aaaattccct                                                 20

<210> 4
<211> 22
<212> DNA
<213> Artificial Sequence

<220>
<223> PCR forward primer

<400> 4
tcctcaactc actcttcgag cg                                              22


<210> 5
<211> 21
<212> DNA

<213> Artificial Sequence

<220>
<223> PCR reverse primer

<400> 5
cggtgctctt gaccaaattg g                                                21